# EUROPÄISCHE PATENTSCHRIFT

(11) **EP 1 305 059 B1**
(45) Veröffentlichungstag und Bekanntmachung des Hinweises auf die Patenterteilung: **02.11.2006**
(21) Anmeldenummer: 01980183.6
(22) Anmeldetag: 20.09.2001
(51) Int. Cl.: A61L 27/34, A61L 27/48

(54) **BIOARTIFIZIELLES VERBUNDMATERIAL UND VERFAHREN ZU SEINER HERSTELLUNG**
BIOARTIFICIAL COMPOSITE MATERIAL AND METHOD FOR PRODUCTION THEREOF
MATIERE COMPOSITE BIOARTIFICIELLE ET SON PROCEDE DE PRODUCTION

(30) Priorität: 25.09.2000 DE 10047300
(43) Veröffentlichungstag der Anmeldung: 02.05.2003
(73) Patentinhaber: Steinhoff, Gustav, 18211 Rethwisch (DE); Freier, Thomas, Toronto, ON M4R 1B1 (CA)
(72) Erfinder: Steinhoff, Gustav, 18211 Rethwisch (DE); Freier, Thomas, Toronto, ON M4R 1B1 (CA)
(74) Vertreter: Garrels, Sabine
(86) Internationale Anmeldenummer: PCT/DE2001/003625
(87) Internationale Veröffentlichungsnummer: WO 2002/024242

(56) Entgegenhaltungen:
- WO-A-99/00152
- FR-A- 2 772 769
- US-A- 4 787 900
- US-A- 5 578 314

## Beschreibung

### [Stand der Technik]

Die Erfindung betrifft ein Verfahren zur Herstellung eines bioartifiziellen Verbundmaterials unter Verwendung einer Bindegewebs-Matrix, die mit Polymeren beschichtet wird.

In der Transplantationstechnik wird heute die Transplantation von Haut, Gefäßen und Organen chirurgisch bereits gut beherrscht und ist weit verbreitet. Die Bereitstellung geeigneter Transplantate ist jedoch noch immer schwierig. Die Transplantate kann man nach ihrer Art in drei große Gruppen einteilen. Zunächst gibt es Transplantate bzw. Implantate aus künstlichen Materialien, wie Kunststoffe, Metalle und Keramik, textilen Materialien usw. je nach Verwendung und dadurch sich ergebender Belastung. Als zweites besteht die Möglichkeit, allogenes Material, z.B. Spenderorgane, oder u.U. auch xenogenes Material (tierischen Ursprungs) zu verwenden. In manchen Fällen wird schließlich versucht, körpereigenes Gewebe für eine Transplantation an anderer Stelle zu verwenden.

Aus der US-PS 5336616 ist bereits ein Verfahren zur Zubereitung eines Gewebes auf Collagen-Basis für die Transplantation bekannt, welches mit Hilfe der Schritte: chemische Vorbehandlung und Zellentfernung; Kryobehandlung, Trockenstabilisierung, Trocknung, Rehydrierung und Zellrekonstitution ein bioartifizielles Transplantat mit folgenden Eigenschaften ermöglichen soll: a) es enthält eine extrazelluläre Protein- Matrix, die möglicherweise vom Wirt remodelliert und repariert werden kann, b) es stellt sich eine intakte Membran-Grundlage für die erfolgreiche Wiederbesiedlung mit lebensfähigen Endothel- oder Epithelzellen dar, c) es ruft primär keine Immunantwort beim Wirt hervor, d) es calzifiziert nicht und e) es kann einfach bei Raumtemperatur gelagert und transportiert werden. Das bekannte Verfahren beruht auf dem Konzept, dass als Transplantat eine möglichst "immun-neutrale" Stützgewebs-Matrix gebildet werden soll, die dann vom Transplantations-Empfänger im Körper umgebaut und remodelliert werden kann. Zur Erleichterung dieses Vorgangs ist auch die Möglichkeit anschließender Besiedlung des nach dem Verfahren hergestellten Transplantates vorgesehen.

Nachteil dieses Verfahren ist die hohe Thrombogenizität der Collagenoberfläche und eine Einschränkung der mechanischen Stabilität.

In der WO 9900152 wird ein bioartifizielles empfängerspezifisches Transplantat sowie ein Verfahren zur Herstellung eines bioartifiziellen Transplantats beschrieben, das folgende Schritte umfasst: a) Bereitstellung eines nativen, eine Collagen-Matrix enthaltenden allogenen oder xenogenen Gewebes bzw. Materials, b) Entfernung weitgehend aller antigen-reaktiver Zellen aus der Collagen-Matrix durch schonende Zellentfernung und anschliessendes Spülen mit steriler wässriger Lösung, c) direkte Weiterverarbeitung des zellfrei gemachten, durch die Behandlung unter b) aufgelockerten Materials durch möglichst vollständige Besiedlung mit den jeweils gewünschten autologen Zellen des Empfängers oder mit genetisch modifizierten für den Empfänger angepassten Zellen, wodurch ein unmittelbar einsatzbereites Transplantat erhalten wird.

Dieses Transplantat bringt insofern Probleme mit sich, dass seine Struktur nicht stabil genug ist, um in jedem Fall eine befriedigende Lösung bei der Transplantation zu geben.

In der Veröffentlichung FR 2772769 A sind der Gegenstand der Erfindung Kopplungsprodukte von Elastin bzw Elastinderivaten und Polymeren (z.B. Polyester: Polyethylenterephthalat, Polylactide; Polyamide: Hexamethylendiaminadipinsäure). Die Kopplung erfolgt dabei durch elektrostatische Wechselwirkung über positiv geladene Stickstoffatome. Das bioartifizielle Verbundmaterial wird in diesem Zusammenhang durch das Aufbringen des Elastins und seiner Derivate auf eine Polymermatrix hergestellt. Hierbei wird kein Proteinmatrix mit einem Polymer beschichtet.

Ein weiterer bekannter Stand der Technik (US 4787900) befasst sich mit einer Blutgefäßprothese mit einer inneren und einer äußeren Schicht. Die innere poröse Schicht ist ein kovalent vernetztes Reaktionsprodukt aus Kollagen und einem Aminopolysaccharid. Die Schicht wird in einem Gießprozess hergestellt. Die poröse äußere Schicht ist eine Kollagen-Aminopolysaccarid-Polymer und wird als Dispersion verarbeitet. Es entsteht eine mehrschichtige zylindrische Verbundstruktur für Blutgefäße.

Die Verarbeitung erfolgt aus einer wässrigen Polysaccarid-Lösung, die mit Kollagen versetzt wurde. Andere Polymere sind nicht genannt. Es erfolgt bei der Beschichtung kein Lösungsmittelaustausch und keine Entwässerung der Matrix.

Es wird kein Gewebe verwendet und es wird keine Mischung der synthetischen Matrix mit Gewebe offenbart.

Gegenstand der Erfindung in US 5578314 ist ein Verfahren zur Beschichtung von Zell- oder Gewebetransplantaten mit Alginat-Polymeren. Dabei werden Gewebezellen oder auch viables, biologisch aktives Gewebe mit einer nicht-fibrogenen Alginat-Zusammensetzung überzogen, wobei die Gewebezellen in einer Überzugs-Kapsel eingeschlossen sind. In einer einzelnen Kapsel ist eine einzelne Zelle oder 1 bis 2 Zellen, insbesondere Pankreaszellen vorhanden. Die Implantation erfolgt durch einfache Injektion mit Hilfe einer subkutanen Nadel.

### [Aufgabe der Erfindung]

Die Aufgabe der Erfindung ist es, Transplantate und ein Verfahren zu ihrer Herstellung zur Verfügung zu stellen, die dem natürlichen Material soweit nachmodelliert sind, dass sie gut einwachsen, nach Möglichkeit zu keinen Abstoßungsreaktionen ("Host-Versus-Graft-reaction") führen, dadurch lange haltbar sind und vom Wirt wie körpereigenes Material aufgefasst und im Rahmen des natürlichen Zellaustausches umgebaut werden, so dass möglichst sogar ein Mitwachsen der Transplantate im Körper des Empfängers ermöglicht wird. Durch ein Beschichtungsverfahren soll die Stabilität und die Bioverträglichkeit des Transplantats erhöht werden.

Die Lösung dieses Problems erfolgt erfindungsgemäß durch ein Verfahren zur Herstellung eines bioartifizielles Verbundmaterials zum Gewebe- oder Organersatz. Dazu wird ein allogenes, xenogenes oder autologes Gewebe, welches eine Bindegewebs-Matrix enthält, die zellfrei ist bereitgestellt.

Die Weiterverarbeitung des Materials erfolgt durch Beschichtung mit Polymeren. Das erhaltene Matrix-Polymer-Verbundmaterial wird in steriler Lösung oder im Festzustand gelagert.

Die Polymere sind bioresorbierbar oder nichtresorbierbar.

Die Beschichtung mit Polymeren erfolgt durch Tauchen in eine Polymerlösung, anschließendes Entnehmen der Matrix aus der Lösung und Trocknen.

Für die Verarbeitung aus der Lösung mit bioresorbierbaren Polymeren werden als Homopolymere Poly(glycolid), Poly(lactid), Poly(ε-caprolacton), Poly(trimethylencarbonat), Poly(p-dioxanon), Poly(hydroxybuttersäure) und längerkettige Poly(hydroxyalkansäuren) sowie Copolymere auf der Basis der genannten Polymere verwendet, die in reiner Form oder als Blends eingesetzt werden. Für die Lösungsmittel werden als niedrigsiedende Verbindungen Dichlormethan, Trichlormethan, 1,2-Dichlorethan, Trichlorethylen, Dibrommethan, Dioxan, Tetrahydrofuran, Ethylacetat, Aceton, Methylethylketon, Acetonitril, Trifluorethanol, Hexafluoraceton-Sesquihydrat und Hexafluorisopropanol sowohl in reiner Form als auch als Gemisch eingesetzt.

Für die Verarbeitung aus der Lösung mit nichtresorbierbaren Polymeren werden Poly(ethylenterephthalat), Poly(urethan), Silikon, Poly(methacrylat), Poly(sulfon), Poly(ethersulfon), Poly(etheretherketon) oder Poly(carbonat) eingesetzt. Als Lösungsmittel kommen, neben den bei den resorbierbaren Polymeren genannten, zusätzlich Pentan, Hexan und Heptan, Cyclohexan, Benzen, Toluen, Diethylether und Dimethylformamid zur Anwendung.

Die bioresorbierbaren und nichtresorbierbaren Polymere können auch als Gemisch untereinander und/oder mit Protein und/oder mit Polysacchariden eingesetzt werden.

Die Beschichtung mit Polymeren kann durch Tauchen in Polymerschmelze, anschließendes Entnehmen der Matrix aus der Schmelze und Erkalten erfolgen.

Für die Verarbeitung aus der Schmelze werden als resorbierbare Polymere Poly(ε-caprolacton), Poly(hydroxyalkansäuren) und Poly(p-dioxanon) mit Schmelzpunkten unter 150 °C oder Copolymere auf der Basis von Poly(ε-caprolacton), Poly(hydroxyalkansäuren) und Poly(p-dioxanon) sowie ausgehend von diesen mit Poly(glycolid) und Poly(lactid) mit Schmelzpunkten unter 150 °C angewendet.

Die verwendete Lösung oder Schmelze kann neben dem Polymer auch Additive enthalten, die Weichmacher, Röntgenkontrastmittel oder Wirkstoffe sind, welche in gelöster Form oder als Suspension vorliegen.

Durch die Zahl der Tauchvorgänge und die Lösungs- oder Schmelzviskosität wird die erforderliche Schichtdicke eingestellt.

Zur Beschichtung können auch Sprühtechniken sowohl aus der Polymerlösung als auch aus der Polymerschmelze angewendet werden.

Das bioartifizielle Verbundmaterial zum Gewebe- oder Organersatz besteht aus einer allogenen, xenogenen oder autologen Bindegewebs-Matrix, welche zellfrei und mit Polymeren beschichtet ist.

Das oben genannte bioartifizielle Verbundmaterial findet als Weichteilgewebeersatz in verschiedenen Organsystemen Anwendung:
a) Haut und Unterhautgewebe,
b) Blutgefäße, Herzgewebe, Herzklappen,
c) Verdauungssystem,
d) Urogenitalsystem,
e) Atmungssystem,
f) Parenchymatöse Organe,
g) Muskel, Bänder und Sehnengewebe,
h) neuronaler Ersatz und periphere Nerven,
i) künstliche extrakorporale und intrakorporale Gefäßsysteme,
k) in vitro Rekonstitution von Organen und Organteilen.

Das nach dem Verfahren erhaltene Verbundmaterial ist unmittelbar einsetzbar. Lagerung und Transport sollte höchst schonend unter sterilen Bedingungen erfolgen.

### [Beispiele]

### Matrixmaterialien

Im folgenden wird die Erfindung anhand von Beispielen beschrieben, die Isolationsprotokolle für die Isolation autologer Zellen und Beispiele für die Durchführung des Verfahrens umfassen.

Als Ausgangsmaterial dient ein allogenes, xenogenes oder autologes Material, das die Grundstruktur für das gewünschte Gewebe, Gefäß oder Organ zur Verfügung stellen soll. Im Einzelfall kann hier auch ein autologes Material verwendet werden, das dem späteren Transplantat-Empfänger zuvor entnommen wurde. Das Ausgangsmaterial wird zellfrei für die Beschichtung eingesetzt.

Erfindungsgemäß soll die Azellularisierung dieses Ausgangsmaterials ausschließlich durch schonende Zellentfernung und anschließendes, ggf. reichliches Spülen mit steriler wässriger Lösung erfolgen. Die schonende Zellentfernung kann entweder durch schonende enzymatische Zellverdauung, beispielsweise durch Einlegen des Gewebes bzw.

Materials in Trypsin-Lösung, erfolgen oder alternativ mit Hilfe eines chemisch zellablösenden Mittels, vorzugsweise mit Hilfe eines ionenspezifischen Komplexbildners.

Im Falle der enzymatischen Zellablösung werden durch das Verdauungsenzym Bindungsstellen in der Verankerung der Zellen mit ihrer Umgebung gelöst. Dies geschieht vorzugsweise durch Einlegen des Gewebes bzw. Materials in Trypsin-Lösung. Dieser Trypsin-Lösung kann bei Bedarf EDTA, EGTA, Triton oder TNN zugesetzt sein. Durch Einstellung der Zeitdauer, während derer das Enzym auf das Gewebe einwirkt, wird das Maß der Abverdauung gesteuert und es wird vermieden, dass ein Angriff auf die Collagen-Matrix selbst erfolgt. Die Trypsinbehandlung bewirkt auch eine gute Auflockerung der zellfrei gemachten Matrix, so dass die Neubesiedlung erleichtert wird.

Alternativ wird ein chemisch zellablösendes Mittel verwendet, das auf beliebige andere chemische Weise die Zellen an ihrer Verankerung zur Collagen-Matrix löst. Vorzugsweise ist vorgesehen, dass ein ionenspezifischer Komplexbildner verwendet wird, der den Zellen essentielle Ionen entzieht und so eine Ablösung verursacht. Durch die Komplexierung z.B. von Calziumionen wird die Bindung der Zellen untereinander und die Zell-Matrixbindung über Integrine aufgehoben.

Als ionenspezifischer Komplexbildner kann beispielsweise das calziumspezifische EGTA (Ethylenglykol- bis -(2-aminoethylether)-tetraessigsäure) eingesetzt werden. EGTA wird vorzugsweise kurzzeitig und hochkonzentriert eingesetzt. Auch andere Komplex- bzw. Chelatbildner, wie EDTA (Ethylendiamintetraessigsäure), Citrat oder Ethylendiamin oder sonstige chemisch zellabtötende und ablösende Mittel, die die Collagen-Proteinstruktur nicht angreifen, können verwendet werden. So bieten sich weiterhin an: BAPTA/AM (ein zellpermeabler Calzium-Chelator), DMSO, Gadolinium, Desferrioxamin, Desferrithiocin, Hexadentat oder auch Aminocarboxylat.

Ein Vorteil der chemisch-mechanischen Behandlung liegt darin, dass der Behandlungsschritt, in dem die Collagen-Matrix zellfrei gemacht wird, nur deutlich weniger Zeit in Anspruch zu nehmen braucht als bei einer Behandlung mit Trypsin. Hierdurch sinkt erstens die Gefahr, dass die Collagen-Matrix selbst angegriffen wird, und zweitens bleibt die Tertiärstruktur besser erhalten, wenn das Substrat nicht zu lange in Lösung gequollen wird. Ein weiterer Vorteil besteht darin, dass durch den Einsatz "harter Chemikalien" Probleme durch u.U. kontaminierte Enzyme tierischen Ursprungs vermieden werden können. Ein weiterer Vorteil liegt schließlich darin, dass die Basalmembran intakt bleibt. Die Basalmembran stellt das direkte und gewebespezifische Adhäsionssubstrat für die Endothelzellen dar. Der Glycosilierungsgrad der Matrixproteine beeinflusst auch die Zellimmigration und somit ein späteres Einwandern von Zellen. Die natürliche Form des Collagengerüstes in ihrer dreidimensionalen Verbindung mit anderen Matrixkomponenten und Integrinen kann daher durch die Erfindung weitgehend erhalten werden.

Auf die beschriebene Weise wird eine aufgelockerte, jedoch in ihrer Sekundär- und Tertiärstruktur dem nativen polymerisierten Collagen noch weitgehend entsprechende Collagenstruktur erhalten, die für das Einwachsen neuer Zellen, nämlich autologer Zellen des Empfängers oder, falls dies im Einzelfall möglich ist, anderer immuntoleranter Zellen bestens vorbereitet ist.

Als Matrixmaterialien können ebenfalls natürliche oder synthetische Proteine/Peptide oder Polysaccharide eingesetzt werden. Geeignete Vertreter der Proteine/Peptide sind z.B. Collagen, Gelatine, Elastin, Fibronektin, Laminin, Polyaminosäuren als Homo- oder Copolymere sowie oligomere Peptideinheiten. Von den Polysacchariden kommen z. B. Cellulose, Hemicellulosen, Pektin, marine Polysaccharide wie Alginat oder Carrageenan, mikrobielle Polysaccharide wie Gellan oder Pullulan, Glycosaminoglycane wie Hyaluronsäure oder Dermatansulfat, Chitin/Chitosan und Speicher-Polysaccharide wie Stärke oder Dextran und darüber hinaus Derivate der genannten Verbindungen als Matrixmaterialien in Frage.

Die Proteine/Peptide und Polysaccharide können durch bekannte Techniken in eine anforderungsgerechte Matrixform überführt werden. Die mechanischen Eigenschaften der Matrix können durch chemische Vernetzungsreaktionen den Erfordernissen angepasst werden. Weiterhin ist es möglich, Mischungen aus Proteinen/Peptiden oder/und Polysacchariden einzusetzen. Den Proteinen/Peptiden oder/und Polysacchariden können Additive zugesetzt werden, wie z.B. synthetische resorbierbare/nichtresorbierbare Polymere, mikrobielle Polyester, Weichmacher, Röntgenkontrastmittel oder Wirkstoffe.

Proteine/Peptide und Polysaccharide können ebenfalls zur Vorbehandlung der Gewebematrix eingesetzt werden, indem diese mit Lösungen oder Suspensionen von Proteinen/Peptiden oder/und Polysacchariden getränkt wird. Auch hier sind die Vernetzung und der Zusatz von Additiven möglich.

### Beschichtungsmaterialien

Für die Beschichtung der Matrixmaterialien kommen je nach Anforderung sowohl resorbierbare als auch nichtresorbierbare Polymere in Betracht. Voraussetzung für die Beschichtung der Gewebe-, Protein- oder Polysaccharidmatrix ist a) für die Verarbeitung aus der Lösung die Löslichkeit des zum Beschichten verwendeten Polymers in einem geeigneten Lösungsmittel oder b) für die Verarbeitung aus der Schmelze eine geeignete Schmelztemperatur. Sowohl die Beschichtung aus der Polymerlösung als auch die Beschichtung aus der Polymerschmelze darf zu keiner Änderung der chemischen Struktur von Beschichtungs- und Matrixmaterial führen, die die biologische Wirksamkeit beeinträchtigt.

Von den resorbierbaren Polymeren, ausgenommen Proteinen/Polypetiden und Polysacchariden, eignen sich für die Verarbeitung aus der Lösung Homopolymere wie z. B. Poly(glycolid), Poly(lactid), Poly(ε-caprolacton), Poly(trimethylencarbonat), Poly(p-dioxanon), Poly(hydroxybuttersäure) und längerkettige Poly(hydroxyalkansäuren) sowie Copolymere auf der Basis der genannten Polymere. Die Polymere und Copolymere können in reiner Form oder als Blends eingesetzt werden. Weiterhin ist der Zusatz von Additiven wie Weichmachern, Röntgenkontrastmitteln oder Wirkstoffen möglich.

Als Lösungsmittel für resorbierbare Polymere können niedrigsiedende Verbindungen wie z. B. Dichlormethan, Trichlormethan, 1,2-Dichlorethan, Trichlorethylen, Dibrommethan, Dioxan, Tetrahydrofuran, Ethylacetat, Aceton, Methylethylketon, Acetonitril, Trifluorethanol, Hexafluoraceton-Sesquihydrat und Hexafluorisopropanol sowohl in reiner Form als auch als Gemisch eingesetzt werden.

Generell besteht die Möglichkeit, durch eine Temperaturänderung das Abdampfverhalten des Lösungsmittels während des Beschichtungsprozesses zu beeinflussen.

Für die Beschichtung aus der Schmelze eignen sich resorbierbare Polymere wie z. B. Poly(ε-caprolacton), Poly(hydroxyalkansäuren) und Poly(p-dioxanon) mit Schmelzpunkten unter 150 °C. Copolymere auf der Basis von Poly(ε-caprolacton), Poly(hydroxyalkansäuren) und Poly(p-dioxanon) sowie ausgehend von diesen mit Poly(glycolid) und Poly(lactid) mit Schmelzpunkten unter 150 °C kommen ebenfalls für die Schmelzeverarbeitung in Betracht. Die maximale Temperatur der Schmelze muss im Einzelfall den Eigenschaften der Matrix angepasst werden. Beim Einsatz von Gewebe- oder Proteinmatrix ist eine Temperatur von 80 °C nicht zu überschreiten.

Prinzipiell ist bei der Lagerung der mit resorbierbaren Polymeren beschichteten Matrizes die Degradationskinetik zu berücksichtigen. Diese kann zu einer begrenzten Haltbarkeit der hergestellten Materialien führen.

Von den nichtresorbierbaren Polymeren, ausgenommen Proteinen/Polypetiden und Polysacchariden, können für die Verarbeitung aus der Lösung z. B. Poly(ethylenterephthalat), Poly(urethan), Silikon, Poly(methacrylat), Poly(sulfon), Poly(ethersulfon), Poly(etheretherketon) und Poly(carbonat) eingesetzt werden. Als Lösungsmittel kommen neben den bei den resorbierbaren Polymeren genannten zusätzlich Verbindungen wie z. B. Pentan, Hexan, Heptan, Cyclohexan, Benzen, Toluen, Diethylether und Dimethylformamid zur Anwendung.

Wiederum besteht die Möglichkeit, durch eine Temperaturänderung das Abdampfverhalten des Lösungsmittels zu beeinflussen. Auch die nichtresorbierbaren Polymere können in reiner Form oder als Blends mit Zusatz von Additiven wie Weichmachern, Röntgenkontrastmitteln oder Wirkstoffen eingesetzt werden.

Für die Beschichtung kommen weiterhin Proteine/Peptide und Polysaccharide in Betracht. Eine stabile Beschichtung erfordert deren Wasserunlöslichkeit. Dafür stehen zum einen in reinem Wasser unlösliche, aber in anderen Lösungsmitteln oder Lösungsmittelgemischen, auch mit Wasser, lösliche Proteine und Polysaccharide zur Verfügung. Zum anderen können wasserlösliche Proteine und Polysaccharide durch eine chemische Nachbehandlung in wasserunlösliche überführt werden. Voraussetzung ist, dass die gewünschten Eigenschaften der Matrix nicht beeinträchtigt werden. Neben milden chemischen Vernetzungsreaktionen, sowohl kovalent als auch ionisch, können z. B. Behandlungen mit verdünnten Säuren oder Basen erfolgen.

Sowohl die resorbierbaren und nichtresorbierbaren Polymere als auch die Proteine und Polysaccharide können als Gemisch für die Beschichtung eingesetzt werden. Hierfür stehen die gleichen Verfahren wie für die Einzelkomponenten zur Verfügung.

### Beschichtungsverfahren

Für die Beschichtung aus der Lösung sind in Abhängigkeit vom verwendeten Matrixmaterial, den Lösungsmitteln und Polymeren unterschiedliche Verfahren erforderlich.

Generell ist eine in wässriger Lösung gelagerte Matrix vor der Beschichtung mit dem zur Beschichtung eingesetzten Lösungsmittel zumindest partiell zu versetzen. Bei der Verwendung von wasserlöslichen Polymeren kann dieser Schritt entfallen. Im Fall von mit Wasser mischbaren Polymerlösungsmitteln ist die Matrix in diesem Lösungsmittel einige Minuten zu lagern und kann anschließend unmittelbar beschichtet werden. Im Fall von mit Wasser nicht mischbaren Lösungsmitteln ist die Matrix zunächst in einem mit Wasser mischbaren Lösungsmittel einige Minuten zu lagern. Anschließend wird in einem Lösungsmittel einige Minuten gelagert, das mit dem vorhergehenden mischbar ist. Diese Prozedur wird wiederholt, bis die Matrix mit dem Polymerlösungsmittel versetzt werden kann. Anschließend kann wieder unmittelbar beschichtet werden. Während des Lösungsmittelaustausches besteht grundsätzlich die Möglichkeit, die Matrix mit Zusatzstoffen, z.B. gelösten Wirkstoffen, zu versetzen.

Im Fall einer trocken gelagerten Matrix ist ein Versetzen mit dem Polymerlösungsmittel nicht erforderlich, es kann direkt beschichtet werden.

Die eigentliche Polymerbeschichtung der Matrix erfolgt durch kurzzeitiges Tauchen in die Polymerlösung, anschließendes Entnehmen der Matrix aus der Lösung und Trocknen. Über die Zahl der Tauchvorgänge und die Lösungsviskosität kann die erforderliche Schichtdicke eingestellt werden. Nach dem Trocknen wird das erhaltene Matrix-Polymer-Verbundmaterial in steriler wässriger Lösung gelagert. Die zum Tauchen verwendete Lösung kann neben dem Polymer auch Additive wie Weichmacher, Röntgenkontrastmittel oder Wirkstoffe enthalten. Diese können in gelöster Form oder als Suspension vorliegen.

Mittels Tauchverfahren ist es möglich, poröse Schichten zu erzeugen, um das Zellwachstum zu beeinflussen. Dazu wird der Polymerlösung ein Additiv zugegeben, das nach der Beschichtung entfernt werden kann, z.B. durch Auswaschen oder thermische Zersetzung. Das Additiv kann in der Polymerlösung in gelöster oder nichtgelöster Form vorliegen. Über das Verhältnis Additiv/Polymer und im Fall unlöslicher Additive über die Größe der Partikel kann die Porosität der zu erzeugenden Verbundmatrix eingestellt werden.

Mittels Tauchverfahren ist es weiterhin möglich, Schichten zu erzeugen, die eine unterschiedliche Zusammensetzung aufweisen. Dadurch können gezielt Wirkstoffe in Kontakt mit dem Matrixmaterial oder dem umgebenden Milieu gelangen oder zu unterschiedlichen Zeiten während des Abbauvorgangs eines resorbierbaren Polymers freigesetzt werden.

Die Beschichtung aus der Polymerschmelze erfolgt in einem Tauchverfahren analog dem für die Polymerlösung beschriebenen. Die Beschichtung der Matrix erfolgt durch kurzzeitiges Tauchen in die Polymerschmelze, anschließendes Entnehmen der Matrix aus der Schmelze und Erkalten. Über die Zahl der Tauchvorgänge und die Schmelzviskosität kann die erforderliche Schichtdicke eingestellt werden. Nach dem Erkalten wird das erhaltene Matrix-Polymer-Verbundmaterial in steriler wässriger Lösung gelagert. Die zum Tauchen verwendete Polymerschmelze kann auch Additive wie Weichmacher, Röntgenkontrastmittel oder Wirkstoffe enthalten.

Mittels Tauchverfahren aus der Polymerschmelze ist es wiederum möglich, poröse Schichten zu erzeugen. Dazu wird der Polymerschmelze ein Additiv zugegeben, das nach der Beschichtung entfernt werden kann. Weiterhin ist es möglich, Schichten zu erzeugen, die eine unterschiedliche Zusammensetzung aufweisen.

Neben dem beschriebenen Tauchverfahren können zur Beschichtung ebenfalls Sprühtechniken sowohl aus der Polymerlösung als auch aus der Polymerschmelze angewendet werden.

### Ausführungsbeispiele

### 1. Matrix

### Matrix aus Poly(β-hydroxybuttersäure)/Gelatine:

0.1 g Gelatine werden in 2.5 ml Ameisensäure gelöst. Anschließend werden 7.5 ml Trifluorethanol zugegeben. Danach erfolgt unter Rühren die Zugabe einer Lösung von 0.4 g Poly(β-hydroxybuttersäure) in 10 ml Trifluorethanol. Die so erhaltene Poly(β-hydroxybuttersäure)/Gelatine-Lösung wird in eine Matrixform gegossen. Nach dem vollständigen Abdampfen der Lösungsmittel wird 30 min mit 1%iger (w/v) wässriger N'-(3-Dimethylaminopropyl)-N-ethylcarbodiimid-Lösung versetzt. Anschließend wird mit Wasser gewaschen und in steriler Pufferlösung gelagert.

### Matrix aus Poly(β-hydroxybuttersäure)/Chitosan:

0.1 g Chitosan werden in 2.5 ml Ameisensäure bis zum vollständigen Quellen gerührt. Anschließend werden 7.5 ml Trifluorethanol zugegeben und bis zum vollständigen Lösen gerührt. Danach erfolgt unter Rühren die Zugabe einer Lösung von 0.1 g Poly(β-hydroxybuttersäure) in 10 ml Trifluorethanol. Die so erhaltene Poly(β-hydroxybuttersäure)/Chitosan-Lösung wird in eine Matrixform gegossen. Nach dem vollständigen Abdampfen der Lösungsmittel wird 30 min mit 1%iger (w/v) wässriger Natronlauge versetzt. Anschließend wird mit Wasser gewaschen und in steriler Pufferlösung gelagert.

### Matrix aus Poly(β-hydroxybuttersäure)/Gelatine/Chitosan:

Je 0.1 g Gelatine und Chitosan werden in 2.5 ml Ameisensäure bis zum vollständigen Quellen gerührt. Anschließend werden 7.5 ml Trifluorethanol zugegeben und bis zum vollständigen Lösen gerührt. Danach erfolgt unter Rühren die Zugabe einer Lösung von 0.1 g Poly(β-hydroxybuttersäure) in 10 ml Trifluorethanol. Die so erhaltene Poly(β-hydroxybuttersäure)/Gelatine/Chitosan-Lösung wird in eine Matrixform gegossen. Nach dem vollständigen Abdampfen der Lösungsmittel wird 30 min mit 1%iger (w/v) wässriger Natronlauge versetzt. Anschließend wird mit Wasser gewaschen und 30 min mit 1%iger (w/v) wässriger N'-(3-Dimethylaminopropyl)-N-ethylcarbodiimid-Lösung versetzt. Nach dem Waschen mit Wasser wird in steriler Pufferlösung gelagert.

### 2. Beschichtung

### Beschichtung von Gewebematrix mit Poly(β-hydroxybuttersäure):

Eine 1.0 x 0.5 cm große Gewebematrix wird 2 x 5 min in Aceton gelagert. Anschließend wird 5 min in Chloroform gelagert. Nach der Chloroformlagerung wird die Gewebematrix in eine 1% (w/v) Lösung von Poly(β-hydroxybuttersäure) in Chloroform getaucht. Nach etwa 1 s wird die Matrix aus der Polymerlösung entnommen und an der Luft getrocknet. Anschließend wird in steriler Pufferlösung gelagert.

### Beschichtung von Gewebematrix mit Gelatine:

Eine 1.0 x 0.5 cm große Gewebematrix wird in eine 1% (w/v) wässrige Gelatinelösung getaucht. Nach etwa 1 s wird die Matrix aus der Proteinlösung entnommen und 30 min mit 1% (w/v) wässriger N'-(3-Dimethylaminopropyl)-N-ethylcarbodiimid-Lösung versetzt. Anschließend wird mit Wasser gewaschen und in steriler Pufferlösung gelagert.

### Beschichtung von Gewebematrix mit Poly(β-hydroxybuttersäure)/Gelatine:

Eine 1.0 x 0.5 cm große Gewebematrix wird 2 x 5 min in Ameisensäure/Trifluorethanol (1:7) gelagert. Danach wird die Gewebematrix in eine 1% (w/v) Lösung von Poly(β-hydroxybuttersäure)/Gelatine (4:1) in Ameisensäure/Trifluorethanol (1:7) getaucht. Nach etwa 1 s wird die Matrix aus der Polymerlösung entnommen und an der Luft getrocknet. Danach wird 30 min mit 1%iger (w/v) wässriger N'-(3-Dimethylaminopropyl)-N-ethylcarbodiimid-Lösung versetzt. Anschließend wird mit Wasser gewaschen und in steriler Pufferlösung gelagert.

### Beschichtung von Gewebematrix mit Chitosan:

Eine 1.0 x 0.5 cm große Gewebematrix wird 2 x 5 min in 1%iger wässriger Essigsäure gelagert. Danach wird die Gewebematrix in eine 1% (w/v) Lösung von Chitosan in 1%iger wässriger Essigsäure getaucht. Nach etwa 1 s wird die Matrix aus der Polysaccharidlösung entnommen und 30 min mit 1%iger (w/v) wässriger Natronlauge versetzt. Anschließend wird mit Wasser gewaschen und in steriler Pufferlösung gelagert.

### Beschichtung von Gewebematrix mit Poly(β-hydroxybuttersäure)/Chitosan:

Eine 1.0 x 0.5 cm große Gewebematrix wird 2 x 5 min in Ameisensäure/Trifluorethanol (1:7) gelagert. Danach wird die Gewebematrix in eine 1%ige (w/v) Lösung von Poly(β-hydroxybuttersäure)/Gelatine (1:1) in Ameisensäure/Trifluorethanol (1:7) getaucht. Nach etwa 1 s wird die Matrix aus der Polymerlösung entnommen und an der Luft getrocknet. Danach wird 30 min mit 1%iger (w/v) wässriger Natronlauge versetzt. Anschließend wird mit Wasser gewaschen und in steriler Pufferlösung gelagert.

### Tauchen aus der Schmelze:

Eine 1.0 x 0.5 cm große Gewebematrix wird in eine auf 60°C erwärmte Poly( 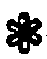 -hydroxybuttersäure)-Schmelze getaucht. Nach etwa 1s wird die Matrix aus der Polymerschmelze entnommen und bis zum Erkalten und Erstarren an der Luft gelagert. Danach wird in steriler Pufferlösung gelagert.

## Patentansprüche

1. Verfahren zur Herstellung eines bioartifiziellen Verbundmaterials, bestehend aus einer Matrix und Polymeren **gekennzeichnet dadurch, dass**
a) ein allogenes, xenogenes oder autologes Gewebe, welches eine Bindegewebs-Matrix enthält, die zellfrei ist, bereitgestellt wird, und
b) die Weiterverarbeitung des Materials durch Beschichtung mit Polymeren erfolgt,
c) das erhaltene Matrix-Polymer-Verbundmaterial in steriler Lösung oder im Festzustand gelagert wird.

2. Verfahren nach Anspruch 1, **gekennzeichnet dadurch, dass** die Polymere bioresorbierbar oder nichtresorbierbar sind.

3. Verfahren nach Anspruch 2, **gekennzeichnet dadurch, dass** die Beschichtung mit Polymeren durch Tauchen in eine Polymerlösung, anschließendes Entnehmen der Matrix aus der Lösung und Trocknen erfolgt.

4. Verfahren nach Anspruch 3, **gekennzeichnet dadurch, dass** für die Verarbeitung aus der Lösung mit bioresorbierbaren Polymeren als Homopolymere Poly(glycolid), Poly(lactid), Poly(ε-caprolacton), Poly(trimethylencarbonat), Poly(p-dioxanon), Poly(hydroxybuttersäure) und längerkettige Poly(hydroxyalkansäuren) sowie Copolymere auf der Basis der genannten Polymere verwendet werden, die in reiner Form oder als Blends eingesetzt werden.

5. Verfahren nach Anspruch 3 oder 4, **gekennzeichnet dadurch, dass**
für die Lösungsmittel als niedrigsiedende Verbindungen Dichlormethan, Trichlormethan, 1,2-Dichlorethan, Trichlorethylen, Dibrommethan, Dioxan, Tetrahydrofuran, Ethylacetat, Aceton, Methylethylketon, Acetonitril, Trifluorethanol, Hexafluoraceton-Sesquihydrat und Hexafluorisopropanol sowohl in reiner Form als auch als Gemisch eingesetzt werden.

6. Verfahren nach Anspruch 3, **gekennzeichnet dadurch, dass** für die Verarbeitung aus der Lösung mit nichtresorbierbaren Polymeren Poly(ethylenterephthalat), Poly(urethan), Silikon, Poly(methacrylat), Poly(sulfon), Poly(ethersulfon), Poly(etheretherketon) oder Poly(carbonat) eingesetzt werden, wobei als Lösungsmittel, neben den bei den resorbierbaren Polymeren genannten, zusätzlich Pentan, Hexan, Heptan, Cyclohexan, Benzen, Toluen, Diethylether und Dimethylformamid zur Anwendung kommen.

7. Verfahren nach Anspruch 3, **gekennzeichnet dadurch, dass** die in den Ansprüchen 4 und 6 verwendeten Polymere als Gemisch untereinander und/oder mit Protein und/oder mit Polysacchariden eingesetzt werden.

8. Verfahren nach Anspruch 2, **gekennzeichnet dadurch, dass** die Beschichtung mit Polymeren durch Tauchen in eine Polymerschmelze, anschließendes Entnehmen der Matrix aus der Schmelze und Erkalten erfolgt.

9. Verfahren nach Anspruch 8, **gekennzeichnet dadurch, dass** für die Verarbeitung aus der Schmelze als resorbierbare Polymere Poly(ε-caprolacton), Poly(hydroxyalkansäuren) und Poly(p-dioxanon) mit Schmelzpunkten unter 150 °C oder Copolymere auf der Basis von Poly(ε-caprolacton), Poly(hydroxyalkansäuren) und Poly(p-dioxanon) sowie ausgehend von diesen mit Poly(glycolid) und Poly(lactid) mit Schmelzpunkten unter 150 °C angewendet werden.

10. Verfahren nach einem der Ansprüche 1 bis 9, **gekennzeichnet dadurch, dass**
die verwendete Lösung oder Schmelze neben dem Polymer auch Additive enthält, die Weichmacher, Röntgenkontrastmittel oder Wirkstoffe sind, welche in gelöster Form oder als Suspension vorliegen.

11. Verfahren nach einem der Ansprüche 1 bis 10, **gekennzeichnet dadurch, dass**
durch die Zahl der Tauchvorgänge und die Lösungs- oder Schmelzviskosität die erforderliche Schichtdicke eingestellt wird.

12. Verfahren nach einem der Ansprüche 1 bis 2, 4 bis 7 oder 9 bis 11, **gekennzeichnet dadurch, dass**
zur Beschichtung Sprühtechniken sowohl aus der Polymerlösung als auch aus der Polymerschmelze angewendet werden.

13. Bioartifizielles Verbundmaterial zum Gewebe- oder Organersatz bestehend aus einer Matrix und Polymeren, **gekennzeichnet dadurch, dass**
das Verbundmaterial aus einem allogenen, xenogenen oder autologen Gewebe besteht, welches eine Bindegewebs-Matrix enthält, die zellfrei ist und mit Polymeren beschichtet ist.

14. Bioartifizielles Verbundmaterials entsprechend der vorhergehenden Patentansprüche 1 bis 13 zur Anwendung als Weichteilgewebeersatz in verschiedensten Applikationen in verschiedenen Organsystemen wie:
a) Haut und Unterhautgewebe,
b) Blutgefäße,
c) Herzgewebe,
d) Herzklappen,
e) Verdauungssystem,
f) Urogenitalsystem,
g) Atmungssystem,
h) Parenchymatöse Organe,
i) Muskel, Bänder und Sehnengewebe,
j) neuronaler Ersatz und periphere Nerven,
k) künstliche extrakorporale und intrakorporale Gefäßsysteme,
l) in vitro Rekonstitution von Organen und Organteilen.

## Claims

1. A method for the production of a biologically artificial composite material consisting of a matrix and polymers, **characterized in that**
a) an allogenic, xenogenic or autologous tissue is provided which contains a connective tissue matrix that is cell-free, and
b) further processing of the material is accomplished by coating with polymers,
c) said obtained matrix polymer composite material is stored in a sterile solution or in the solid state.

2. A method according to claim 1, **characterized in that** said polymers are bioresorbable or not resorbable.

3. A method according to claim 2, **characterized in that** said coating with polymers is accomplished by dipping into a polymer solution, subsequently removing said matrix from said solution, and drying.

4. A method according to claim 3, **characterized in that** for said processing from said solution with bioresorbable polymers polyglycolide, polylactide, poly(ε-caprolactone), polytrimethylen carbonate, poly(p-dioxanone), polyhydroxy butyric acid and longer-chain polyhydroxy alkanoates, as well as copolymers based on said mentioned polymers which are applied in a pure form or as blends are used as homopolymers.

5. A method according to claim 3 or 4, **characterized in that** dichloromethane, trichloromethane, 1,2-dichloroethane, trichloroethylene, dibromomethane, dioxane, tetrahydrofuran, ethyl acetate, acetone, methyl ethyl ketone, acetonitrile, trifluoroethanol, hexafluoroaceton-sesquihydrate and hexafluoroisopropanol, both in a pure form as well as in a mixture are used as low-boiling compounds for said solvents.

6. A method according to claim 3, **characterized in that** for processing from said solution with non-resorbable polymers polyethylene terephthalate, polyurethane, silicone, polymethacrylate, polysulfone), polyether sulfone, polyether ether ether ketone or polycarbonate) are applied, wherein additionally pentane, hexane, heptane, cyclohexane, benzene, toluene, diethyl ether, and dimethyl formamide are used as solvents in addition to those solvents mentioned with said resorbable polymers.

7. A method according to claim 3, **characterized in that** said polymers, as used in claims 4 and 6, are applied as a blend of one another, and/or with protein and/or with polysaccharides.

8. A method according to claim 2, **characterized in that** said coating with polymers is accomplished by dipping into a polymer melt and subsequently removing said matrix from said melt, and cooling down.

9. A method according to claim 8, **characterized in that** for said processing from said melt poly(ε-caprolactone), polyhydroxy alkanoates and poly(p-dioxanone) having melting points below 150°C, or copolymers based on poly(ε-caprolactone), polyhydroxy alkanoates and poly(p-dioxanone), as well as resulting therefrom with polyglycolide and polylactide having melting points below 150°C are used as resorbable polymers.

10. A method according to any one of the claims 1 to 9, **characterized in that** said used solution or melt also include additives, in addition to the polymer, which are either softeners, X-ray contrast media, or active substances that are present in a dissolved form or as a suspension.

11. A method according to any one of the claims 1 to 10, **characterized in that** the required layer thickness is adjusted by the number of dipping actions and the solution or melting viscosities.

12. A method according to any one of the claims 1 to 2, 4 to 7, or 9 to 11, **characterized in that** spraying techniques from both said polymer solution and said polymer melt can be applied for coating.

13. A bioartificial composite material for tissue and organ replacement, consisting of a matrix and polymers, **characterized in that** said composite material consists of an allogenic, xenogenic, or autologous tissue which contains a connective tissue matrix that is cell-free, and is coated with polymers.

14. A bioartificial composite material according to the preceding claims 1 to 13 for the use as a soft-tissue replacement in most various applications in different organ systems such as:
a) skin and subskin tissues
b) blood vessels
c) heart tissues,
d) heart valves
e) digestive system
f) urogenital system
g) respiratory system
h) parenchymatosous organs
i) muscles, ligaments and tendon tissues
j) neuronal replacement and peripheral nerves
k) artificial extracorporeal and intracorporeal vessel systems
l) in-vitro reconstitution of organs and organ portions.

## Revendications

1. Procédé pour la réalisation d'un composé bioartificiel consistant d'une matrice et de polymères, **caractérisé en ce que**
a) un tissu allogène, xenogène ou autologe contenant une matrice de tissu conjonctif non-cellulaire soit mise à la disposition et
b) la transformation du matériel s'éffectue par le revêtement de polymères
c) le composé de matrice polymérique sera entreposé en solution stérile ou à l'état solide.

2. Procédé selon la revendication 1, **caractérisé en ce que** les polymères sont resorbables biologiquement ou non-resorbables.

3. Procédé selon la revendication 2, **caractérisé en ce que** le revêtement de polymères s'effectue par l'immersion dans une solution polymérique, suivi par l'enlèvement de la matrice et par son sêchage.

4. Procédé selon la revendication 3, **caractérisé en ce que** pour la transformation de la solution avec des polymères bioresorbables comme homopolymères est utilisé du poly (glycocide), poly (lactide), poly (e-caprolactène), poly (carbonate triméthylénique), poly (p-dioxanone), de l'acide poly (hydroxibutyrique), et des acides poly (hydroxyalcanes) en chaînes longues ainsi que des copolymères sur la base desdites polymères, utilisés en état pure ou en blends.

5. Procédé selon les revendications 3 ou 4, **caractérisé en ce que** pour les solutions comme composés à température d'ébullition basse est utilisé du méthane dichlorique, du méthane trichlorique, du dioxane, du furane tetrahydrique, de l'acétate éthylique, de l'acétone, de l'acétone méthy-éthylique, du nilryle acétonique, de l' éthanole trifluorique, de l'acétone hexafluorique-sesquihydrate, et de l'isopropanole hexafluorique non seulement en état pure mais encore en mélange.

6. Procédé selon la revendication 3, **caractérisé en ce que** pour la transformation de la solution aves des polymères on utilise des térephtalates polyéthyléniques, des poly (uréthanes), du silicône, des poly (méthacrylates), des poly (sulphones), des poly (éthersulphones), poly (éthèreéthèrecétone) ou poly (carbonates), tandis que comme dissolvant sont appliqués du pentane, hexane, heptane, cyclohexane, benzène, tuolène, éthère diéthylique, formamide diméthylique, sans compter ceux qui étaient mentionnés avec les polymères resorbables.

7. Procédé selon la revendication 3, **caractérisé en ce que** les polymères utilisés dans les revendications 4 et 6 sont utilisés comme mélange entres eux et/ou utilisés avec des protéines et/ou des polysaccarides.

8. Procédé selon la revendication 2, **caractérisé en ce que** le revêtement de polymères s'effectue par l'immersion dans une fusion de polymères, suivi par l'enlèvement de la matrice de la fusion et par son refroidissement.

9. Procédé selon la revendication 8, **caractérisé en ce que** pour la transformation de la fusion comme polymères resorbables est utilisé du poly (e-caprolactone), des acides polyhydroxyalides et du poly (p-dioxanone) avec des points de fusion en dessous 150 C° ou de copolymères sur base de poly (e-caprolactone), des acides polyhydroxyaliques et du poly (p-dioxanone) ainsi que basant desdites polymères avec du poly (glycolide) et poly (lactide) avec des points de fusion en dessous 150 C°.

10. Procédé selon l'une des revendications 1 à 9, **caractérisé en ce que** la solution ou la fusion utilisé contient non seulement du polymère mais encore des additifs qui sont des plastifiants, des substances contrastés radiologiques ou des agents qui se présentent sous forme soluble ou en suspension.

11. Procédé selon l'une des revendications 1 à 10, **caractérisé en ce qu'**on atteint l'épaisseur de revêtement par le nombre des immersions et par la viscosité de la solution ou de la fusion.

12. Procédé selon l'une des revendications 1 à 2, 4 à 7 ou 9 à 11, **caractérisé en ce que** pour le revêtement sont mis en oeuvre des procédés de vaporisation non seulement avec la solution polymérique mais aussi avec la fusion polymérique.

13. Composé bioartificiel pour le remplacement de tissu ou d'organes consistant d'une matrice de polymères, **caractérisé en ce que** le composé consiste d'un tissu allogène, xénogène ou autologe, contenant une matière de tissu conjonctif non-cellulaire et revêtée de polymères.

14. Composé bioartificiel selon les revendications précédentes 1 à 13 pour l'application comme ersatz de tissu de parties molles dans des différentes applications dans des différentes systèmes d'organes comme
a) de tissu de peau et de peau sous-cutané
b) des vaisseaux sanguins
c) de tissu cardiaque
d) des valvules de coeur
e) des organes de digestion
f) des organes urogénitaux
g) le système respiratoire
h) des organes parenchymatoires
i) des muscles, des tendons et des tissus de tendon
j) ersatz neuronale et nerfs périphères
k) systèmes vasculaires artificiels extracorporales et intracorporales
l) reconstitution des organes et des parties d'organes.
